# EUROPEAN PATENT SPECIFICATION

(11) **EP 3 695 791 B1**
(45) Date of publication and mention of the grant of the patent: **02.04.2025**
(21) Application number: 18865903.1
(22) Date of filing: 30.08.2018
(51) Int. Cl.: A61B 17/02, A61M 25/10, A61M 25/00, A61M 25/01, A61B 90/00

(54) **TRACTION DEVICE FOR CURVED BALLOON CATHETER**
ZUGVORRICHTUNG FÜR EINEN GEKRÜMMTEN BALLONKATHETER
DISPOSITIF DE TRACTION POUR CATHÉTER À BALLONNET INCURVÉ

(30) Priority: 10.10.2017 CN 201710938901
(43) Date of publication of application: 19.08.2020
(73) Proprietor: Shanghai Techbank Medical Technology Co., Ltd., Shanghai 200092 (CN)
(72) Inventor: ZHANG, Chuanhai, Shanghai 200135 (CN); SUBBAKRISHNA, Shankar, Shanghai 200135 (CN); LI, Fanqi, Shanghai 200135 (CN)
(74) Representative: Cameron Intellectual Property Ltd
(86) International application number: PCT/CN2018/103280
(87) International publication number: WO 2019/072047

(56) References cited:
- EP-A2- 0 521 595
- WO-A1-2017/004432
- WO-A1-2017/049313
- WO-A2-2014/096370
- CN-A- 103 442 650
- CN-A- 104 921 853
- CN-U- 2 120 592
- CN-U- 204 581 372
- CN-Y- 201 333 252
- US-A1- 2002 165 537
- US-A1- 2014 243 580
- US-A1- 2014 277 062
- US-A1- 2015 202 089
- US-A1- 2017 252 027
- US-A9- 2017 150 957

## Description

### TECHNICAL FIELD

The invention relates to a catheter retractor, in particular to a curved balloon catheter retractor device.

### BACKGROUND OF THE INVENTION

Retractors, also known as retractors, are used to retract tissues, reveal the surgery space, and facilitate exploration and operation. They can be divided into handheld retractors and automatic retractors. There are various specifications of different shapes and sizes, and the appropriate retractor can be selected according to the needs of the operation.

The traditional retractor requires larger operating space, which leads to a larger surgical wound. At the same time, traditional retractors are mostly made of metal with sharp ends, which are likely to cause secondary trauma to the patient and damage important organs and tissues.

At present, the adjustable tissue retractor is gradually being widely used, and it can pass through the working lumen of the adjustable endoscope. Tissue retractors are used in endoscopy and open surgery, including endoscopy, laparoscopy and general surgery. In order to meet the specific requirements of surgical operations, the length and diameter of this tissue retractor can be fixed or adjustable . The adjustable endoscopic tissue retractor can be used to fix the organ tissue so that it can be retracted and manipulated in a certain way.

The catheter retractor is used for tissue retraction in surgery, and the product is used to facilitate the retraction operation through natural cavity or open surgical intervention. Surgery includes, but is not limited to, various types of laparoscopic surgery, cardiovascular surgery, brain surgery, gastrointestinal surgery, urinary disease surgery, etc. The tissues to be retracted include but are not limited to gastrointestinal tract, esophagus, airway, urethra, vagina, bladder, etc. The purpose of retraction includes but is not limited to protecting specific tissues and removing specific tissues to facilitate surgical operations.

Using the balloon to expand and deform to create space between the tissues is a better solution than the metal retractor. It is small in size and can be placed in the human tissue that needs to be isolated without excessively damaging the human tissue. In the meanwhile, the surface of the expanded balloon is smooth and soft, and it is not easy to cause damage to human tissues. For example, Chinese Patent 200580028684.5 provides a tissue removal/separation device. The device includes a balloon that can be inflated between a first tissue and a second tissue of the body. The balloon has an expanded shape that can be selected to remove or separate the first tissue from the second tissue in a manner suitable to protect the first tissue from the application of treatment to the second tissue.

However, this invention only reveals the possibility of using balloon inflation to isolate human tissues. An original inflatable balloon is not suitable for inflation and isolation in any human tissues. Even to a certain extent, the use of such a balloon to inflate in the human body will cause a huge potential risk. Moreover, when inflated to a certain pressure, the balloon will closely adhere to the human tissue. Although it may isolate different tissues, it may also tightly fills the limited space among the tissues, making the operation more difficult. If sharp surgical devices and heat treatment are used, the balloon may burst. Therefore, compared with a mechanical retractor, the balloon inflation device cannot effectively achieve significant deviation distance and at the same time does not occupy operation space. It can only play a role in isolating human tissues. In addition, it is difficult to determine the specific location of the balloon from outside the body. And in general, this type of surgery is usually quite precise. At the same time, fluid accumulation in the body is difficult to discharge, leading to new risks.

Therefore, there is a need for a surgical device that utilizes balloon as a retractor that is more comprehensive and suitable for practical surgeries to consolidate the advantages of both balloon inflation and mechanical retraction.

Document US2015342590 A1 discloses a laparoscopic retractor that comprises a shaft having a proximal and distal end, the distal end of the shaft being configured for adjustment from a substantially straight orientation to a curved orientation having a substantially U-shaped hook suitable for laparoscopic retraction. The proximal end of the shaft comprises a handle operably connected to the distal end of the shaft for adjustment of the distal end from the substantially straight orientation to the curved orientation. The distal end of the shaft is enclosed within a flexible sheath that is adapted to be inflated, and wherein a portion of the sheath covering the distal end of the shaft comprises inflatable balloon means such as one or more inflatable balloons which cover the substantially U-shaped hook.

Document US2017252027 A1 discloses systems, devices, components and methods for repositioning or displacing a patient's esophagus a safe distance away from the patient's heart during an atrial ablation surgical procedure. An esophageal displacement catheter is disclosed that is configured to reposition a patient's esophagus 20 mm or more away from the ablation location in the patient's heart. A distendable section of the catheter is configured such that portions of a first pulling member extend sufficiently far away from joints located in the distendable section when the first pulling member is in a retracted position to permit the distendable section to assume a deployed and distended configuration. At least one flexible deformable or distendable member, sheath or covering is configured to be disposed over and cover the portions of the first pulling member extending away from the joints when the distendable section is the deployed and distended configuration. One or more balloons can also be incorporated into the catheter to enlarge controllably the diameter of the distendable section.

### SUMMARY OF THE INVENTION

The invention aims to provide a curved balloon catheter retractor device for natural cavity intervention or open surgical intervention based on the above problems. It solves the problem that the current retractor and balloon isolation device are not accurate and reliable enough to adapt to surgery to realize a simple and reliable catheter retraction.

In order to achieve the above objective, the present invention provides a curved balloon catheter retractor as defined in claim 1.

The present disclosure provides as well a curved balloon retractor, which includes a catheter with a handle at one end, a positioner and a retractor balloon arranged on the outside of the catheter, wherein the catheter is provided with one or more lumens, and the catheter part is provided with at least one opening through which the at least one lumen inflates and deflates the traction balloon with fluid. When the traction balloon is filled with fluid, it will bend to one side, and the handle can drive the catheter and the traction balloon to rotate.

Wherein, the positioner can be equipped with radiopaque material, and its position in the body can be observed by X-ray. The positioner is one or a plurality of positioning balloons located at the fixed position of the catheter, and the positioning balloons can be stuck in the cavity of the human body after being filled with fluid. The handle includes a part that can be held and another part that can be rotated. There is an angular dimension on the handle indicating the angle of rotation. When the handle is rotated, it is used to mark the relative azimuth angle of the traction balloon. The catheter is made of a material that is not susceptible to self-twisting; when rotate the part of the catheter that is left outside the body cavity, the traction balloon rotates at a certain proportion of the angle at the same time. There is a length scale on the catheter to mark the depth of catheter insertion. One or more lumens are used to inflate the positioning balloon. At least one of the lumens is used to drain the effusion. The location of the opening of the lumen for draining effusion on the catheter is located near the proximal end of the traction balloon; if there is a positioning balloon, the opening is located near the proximal end of the positioning balloon. There is at least one lumen for spraying the contrast agent; the opening of lumen for spraying the contrast agent is located near the proximal end of the traction balloon; if there is a positioning balloon, it is located between the traction balloon and the upper positioning balloon. There is at least one lumen for sucking negative pressure, and its opening is located between the positioning balloons at both ends, and there can be one or a plurality of openings for sucking negative pressure. The traction balloon can be made of materials mixed with radiopaque substance, so that it can be fully observed under X-rays. The traction balloon is used to retract the esophagus. The traction balloon is positioned between the second and third stenosis of the esophagus. After being inflated with fluid, the diameter of the positioning balloon is larger than the diameter of the esophagus at the stenosis.

Another objective of the present disclosure is to provide a curved balloon catheter retractor, which is used for esophageal retraction in cardiac ablation surgery. In order to achieve this objective, the invention provides a curved balloon catheter retractor including a handle, a multi-lumen catheter, two positioning balloons, and one traction balloon; wherein the three lumens of the multi-lumen catheter are respectively opened at the positions where the three balloons are located in order to inflate the three balloons with fluid respectively; two positioning balloons are located at both ends of the traction balloon, which clamp the esophagus for fixation respectively when inflated with fluid; when the traction balloon is inflated with fluid, it drives the esophagus to bend and pulls it away from its original position.

Whereas the four lumens are positioned as a large lumen in the middle with a plurality of small lumens around it; Each small lumens has an opening at the position of each balloon to inflate the balloon individually; The large lumen in the middle has an opening at the proximal end of the upper positioning balloon to drain saliva. There is another lumen communicates with both the positioning balloon and the traction balloon, and is used deflate fluid from the balloons. There is one more lumen can be used to spray contrast agent to the inner wall of the esophagus to show the curvature of the esophagus.

Another objective of the present disclosure is to provide a safe and effective retraction method for a curved balloon catheter. In order to achieve this objective, the present invention provides a retraction method for a curved balloon catheter, which is characterized by the following steps: Inserting an uninflated curved balloon catheter into the body cavity that needs to be retracted; Monitoring whether the positioning balloon arrives the desired position; Inflate the positioning balloon with fluid to make it stuck in the human body cavity; Inflate the traction balloon with fluid to make it bend to achieve the purpose of retracting the human body cavity.

After the positioning balloon is filled with fluid, it also includes a step of sucking fluid from the cavity by utilizing negative pressure, and then inflating the traction balloon to achieve the purpose of retracting the cavity.

Another objective of the present invention is to provide a curved balloon catheter retraction method for esophageal retraction, which is characterized by including the following steps: Inserting the balloon catheter from the oral or nasal cavity into the esophagus, and when the positioning balloon at the distal end reaches between the second stenosis and the third stenosis, inflate the positioning balloon at the distal end and continue to push it to and above the third stenosis; Inflate the positioning balloon at the proximal end so that it is stuck below the second stenosis; Then confirm the bending direction of the balloon relative to the heart by referring to the markers on the catheter and handle, and inflate the traction balloon in the middle to bend and drive the esophagus away from the heart or cardiac surgery site.

When the bending direction of the traction balloon needs to be changed during use, the catheter and the balloon can be driven to rotate by the rotating the handle, so that the esophagus is driven to rotate, thereby always avoiding the tissues or positions that need to be avoided during the surgery. When the balloon is used to deviate the esophagus, the balloon is located between the second and third stenosis of the esophagus, and the diameter of the positioning balloon after being inflated with fluid is larger than the diameter of the esophagus at the stenosis. When in use, the positioning balloon is first inflated with fluid, and then use negative pressure to suck air in the esophagus, and finally the traction balloon is bended to achieve the purpose of driving the esophagus to bend.

The beneficial effects of the present invention are: the present invention provides a better device for human tissue retraction. Compared with the existing tissue retraction methods, its maneuverability is better, safer, and the patient is more comfortable. Specifically, if it is used for esophageal retraction, the existing way of placing the device through the mouth can be changed to the way of placing it through the nose, which is better to operate. The use of positioning balloon and angular dimension marker makes the tissue retracting operation (point of action and retracting direction) more accurate. The increased lumens and openings enable more practical operations such as saliva drainage, spraying of contrast agent, and suction of negative pressure.

### BRIEF DESCRIPTION OF THE DRAWINGS

FIG. 1 is a schematic view of the structure of the present invention.
FIG. 2 is a cross-sectional view of a catheter portion of the present invention.
FIG. 3 is a schematic view of a handle portion of the present invention.
FIG. 4 is a schematic diagram of one embodiment of the present invention.
FIG. 5 is a schematic diagram of another embodiment of the present invention.
FIG. 6 is a cross-sectional view of the catheter portion of the embodiment of FIG. 5.
FIG. 7 is a schematic view of yet another embodiment of the present invention.
FIG. 8 is a cross-sectional view of the catheter portion of the embodiment of FIG. 7.
FIG. 9 is a schematic drawing of a curved balloon catheter retractor device.

### DETAILED DESCRIPTION OF THE PREFERRED EMBODIMENTS

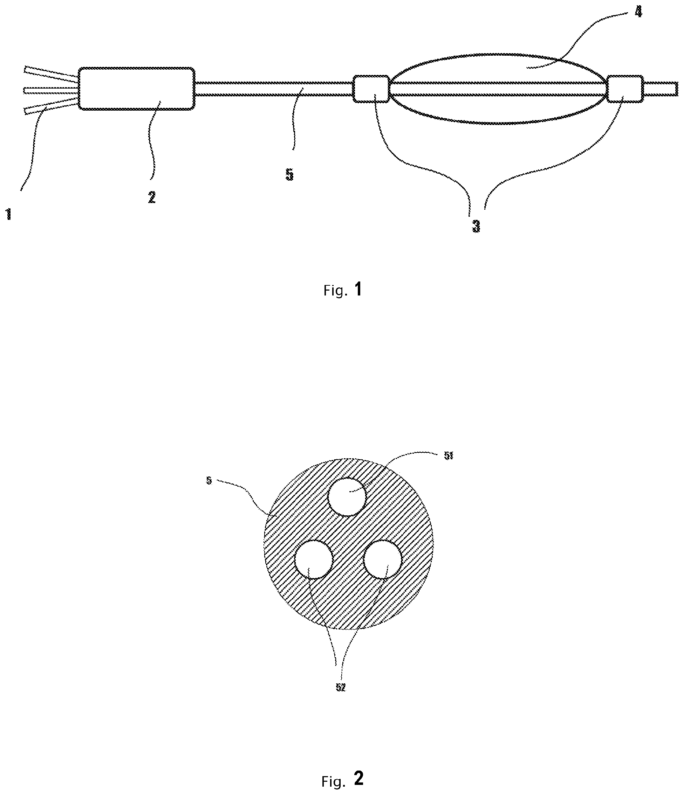

The invention will be described in further detail below in conjunction with the appended drawings and examples:
Please refer to FIG. 1, which is a schematic view of the structure of the present invention. As shown in FIG. 1, curved balloon catheter retractor of the present invention is composed of the following parts: A curved balloon catheter retractor of the present invention is composed of the following parts: a handle 2 with a duct and a valve 1 at one end, a positioning balloon 3 and a traction balloon 4 arranged outside the catheter 5, and a catheter 5 is provided with one or multiple lumens, the portion of catheter 5 within the positioner 3 is provided with at least one opening and at least one lumen controlled by the duct and the valve 1 to inflate and deflate the positioning balloon 3 and a traction balloon 4with fluid. The fluid referred here is usually liquid or gas. When filled with fluid, the traction balloon 4 will bend to one side. In one embodiment, the gas is injected into the gas inlet by a syringe. After injection, block the duct and valve 1 so that the traction balloon 4 can maintain a given pressure. Generally speaking, the traction balloon 4 is located outside the catheter 5 near the middle or distal end. And the upper and lower ends of the traction balloon 4 are sealed on the catheter 5 by laser welding or bonding. The advantage of laser welding is that retraction requires a relatively large pressure inside the balloon to generate sufficient rigidity, sometimes reaching 10 atmospheres. Only laser welding can ensure a reliable seal. The material of the traction balloon 4 of the present invention is semi-compliant or non-compliant, that is to say, the volume and shape of the traction balloon 4 slightly changes in a relatively wide range of pressure, so that the organ won't be excessively squeezed. The positioner 3 can be composed of two compliant positioning balloons 3, which are respectively fixed on the catheters 5 at the proximal and distal end of the traction balloon 4. In some embodiments, there are one or a plurality of positioning balloon 3. In the case of one positioning balloon 3, it can be placed at the proximal or distal end of the traction balloon 4. The positioning balloon 3 can be compliant or non-compliant balloon with a relatively small length to diameter ratio. In the case of a plurality of positioning balloons 3, they can be filled with fluid (gas or liquid) as a whole or individually. The traction balloon 4 is a non-compliant balloon with a relatively length to diameter ratio. The positioning balloon 3 and the traction balloon 4 can be made of developing materials, so that they can be fully developed under X-rays.

Next, please refer to FIG. 2, which is a cross-sectional view of the catheter part of the present invention. In FIG. 2, the cross section of the catheter 5 has three lumens, of which two lumens 52 are used to inflate and deflate the positioning balloon 3, and the other lumen 51 is used to inflate and deflate the traction balloon 4.

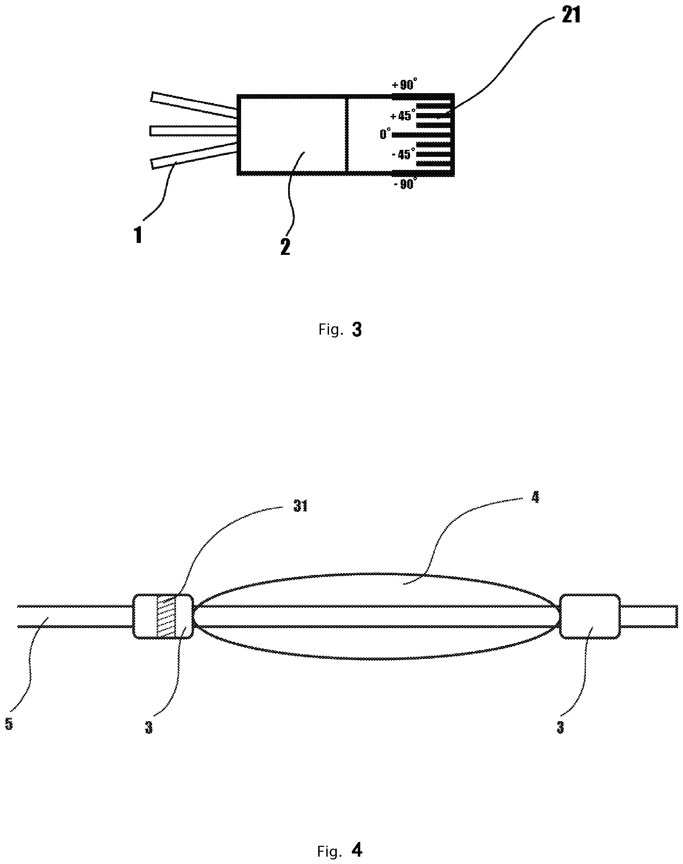

Figure 3 is a schematic diagram of the handle portion of the present invention. The handle 2 is used for operations such as inflation, injection, holding and rotation. There are one or a plurality of ports 1 for inflation and/or injection according to specific design. The handle 2 includes a part that can be held stationary and a part that can be rotated to a certain angle. The handle 2 is provided with an angular dimension 21 indicating the angle. When the handle 2 is rotated, it is used to mark the relative angular position of the traction balloon relative to the organ or the tissue to be retracted.

Next, please refer to FIG. 4, which is a schematic diagram of an embodiment of the present invention. In Fig. 4, there is a positioning marker 31 on the positioner or positioning balloon 3, which can be radiopaque materials, such as a barium sulfate. It can also be other positioning devices such as infrared spectroscopy or RFID, which enable the doctor to detect the specific position of the positioner or positioning balloon 3 in the patient's body from outside. In some embodiments, there is a radiopaque line on the catheter 5, which is used for X-ray imaging.

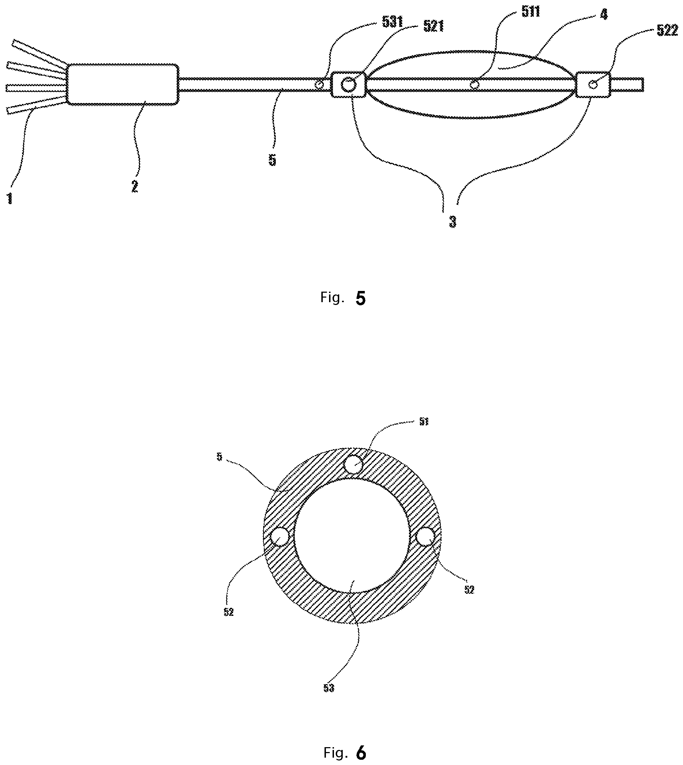

Please refer to FIG. 5 and FIG. 6. FIG. 5 is a schematic diagram of another embodiment of the present invention. Fig. 6 is a cross-sectional view of the catheter part of the embodiment of Fig. 5. The curved balloon catheter retractor includes a handle 2, a four-lumen catheter 5, two compliant positioning balloons 3, and a semi-compliant or non-compliant retraction balloon 4. The positions of the four lumens are the large lumen 53 in the middle with the three small lumens 51 and 52 around it. The three small lumens have openings 511, 521, and 522 at the positions where the three balloons are located in order to inflate the three balloons individually. The large lumen 53 in the middle has an opening 531 near the proximal end of the positioning balloon for draining saliva.

The catheter 5 is a hollow catheter, and there are one or a plurality of lumens inside the catheter 5. At least one of the lumens 51 is dedicated to inflate the traction balloon 4 with fluid. One or a plurality of lumens are used to inflate the positioning balloon 3. Among the lumens, at least one lumen 53 is used to drain saliva; the opening 531 of the saliva draining lumen 53 in the catheter 5 is located near the proximal end of the traction balloon 4; if there is a positioning balloon 3, then the opening 531 is located near the proximal end of the positioning balloon 3.

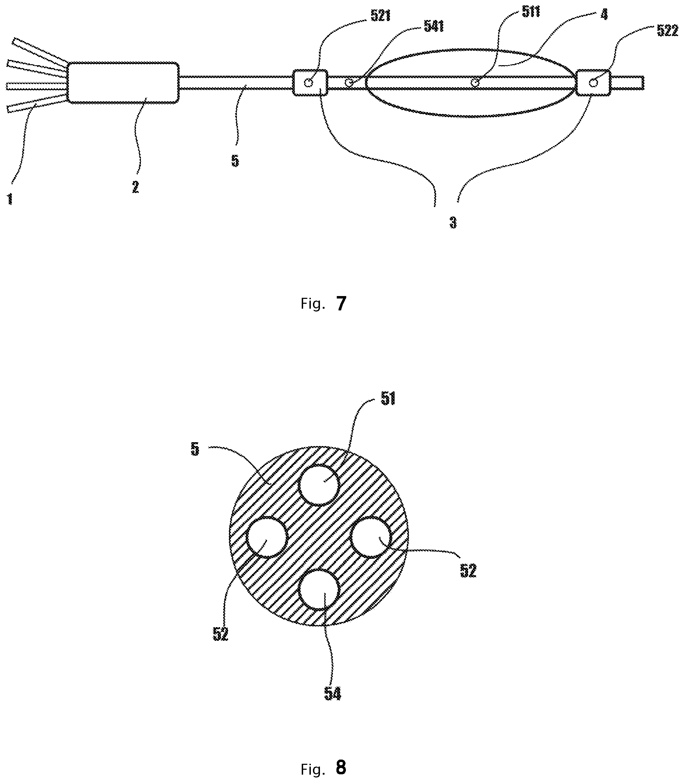

Please refer to FIG. 7 and FIG. 8. FIG. 7 is a schematic diagram of another embodiment of the present invention. Fig. 8 is a cross-sectional view of the catheter part of the embodiment of Fig. 7. Different from the above embodiment, all the lumens in this embodiment have no obvious differences in size. The three lumens 51 and 52 of the four-lumen catheter 5 have individual openings at the positions where the three balloons are located, and are used to inflate the three balloons individually. Another lumen 54 is used to spray the contrast agent (barium meal; barium sulfate); the hole 541 for spraying the contrast agent is located near the proximal end of the traction balloon 4; if there is a positioning balloon 3, the hole 541 is located between the traction balloon 4 and the upper positioning balloon 3. The function of spraying the contrast agent is to make the inner wall of the esophagus visible, and to know the exact deviation distance.

In another embodiment, the lumen 54 is used for suction of negative pressure, and its opening is located between the two positioning balloons 3, and there may be one or a plurality of openings for suction of negative pressure. Specifically, during use, the positioning balloon 3 is inflated first, and then air is drawn from the lumen 54 with negative pressure, and then the traction balloon 4 is inflated to achieve the purpose of bending the esophagus.

The said catheter is made of a material that is not susceptible to self-twisting; when rotating the part of catheter that is left outside the body cavity, the part of catheter inside the body cavity rotates at a certain proportion of the angle at the same time. In the meanwhile, there is a length scale on the catheter to mark the depth of catheter insertion.

The present disclosure also provides a retraction method, of which the single-lumen/multi-lumen catheter can be inserted into the animal body, the traction balloon can be expanded into arc shape after fluid is injected and the traction balloon is attached to the outside of the catheter. The catheter can be offset toward the protruding direction of the traction balloon, and can be equipped with an operating handle and other related accessories for joint use. Used for tissue retraction in surgery, the product can complete the retraction operation through natural cavity intervention or open surgical intervention. Surgery includes, but is not limited to, various types of laparoscopic surgery, cardiovascular surgery, brain surgery, gastrointestinal surgery, urinary disease surgery, etc. The retracted tissues include but are not limited to the gastrointestinal tract, esophagus, airway, urethra, vagina, bladder, etc. The purpose of retraction includes, but is not limited to, protecting specific tissues and moving away specific tissues to facilitate surgical operations.

The curved balloon catheter retractor is inserted into the body through the natural cavity of the human body or the surgical method. The position and rotation angle of the catheter can be adjusted by the handle with the assistance of the imaging equipment, and the position and angle can be locked by the handle. The balloon can be inflated through the injection port attached to the handle or any other injection path. After the balloon is inflated, it expands and assumes a curved shape. At least one section of the catheter will achieve different degrees of bending. Finally, the curved part of the balloon catheter can achieve the retraction or displacement of the tissue.

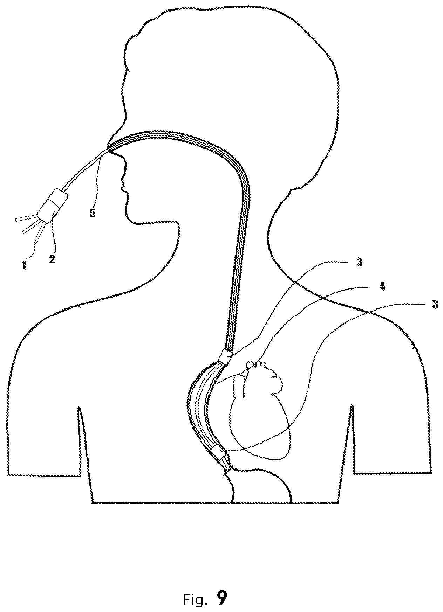

Take the complications of atrial fibrillation ablation as an example to introduce the role of catheter retractors. Atrial fibrillation is the most common arrhythmia, and radiofrequency ablation for atrial fibrillation has gradually been recognized as a better solution than medication and conventional surgery in recent years. Complications of atrial esophageal fistula originate from the spatial relationship between the left atrium and the esophagus. Since the esophagus is located at the most anterior part of the posterior mediastinum, it is only separated from the posterior wall of the left atrium by the oblique sinus of the pericardium. While the posterior wall of the left atrium and the anterior wall of the esophagus are very thin, the high temperature and energy during ablation surgery is very likely to cause excessive damage to the esophagus. The complication of atrial esophageal fistula has a very high mortality rate. In order to make the operation safe, the esophagus must be retracted away from the heart. Figure 9 is a schematic diagram of the esophagus retracted by a curved balloon catheter retractor. In Figure 9, a catheter with a balloon on the outside is inserted into the natural or surgically formed cavity of the patient, and the part of balloon is inserted into the cavity that needs to be retracted. After inflating the balloon through the catheter, the balloon is expanded and bends to one side. And the catheter is pulled to bend and cause the patient's cavity to bend so that the retraction displacement is achieved.

When in use, insert the catheter 5 from the nasal cavity into the esophagus. When the positioning balloon 3 at the distal end enters between the second and third stenosis of the esophagus, inflate the positioning balloon 3 at the distal end and continue to push it to and above the third stenosis; inflate the positioning balloon 3 at the proximal end so that it is stuck below the second stenosis; then inflate the traction balloon 4 in the middle to make it bend and drive the esophagus to bend. During use, the catheter 5 and balloons 3, 4 are driven to rotate by the rotating handle 2, so that the deviation direction of the esophagus in the body is changed, so that the ablation point of cardiac ablation is always avoided. After the positioning balloon 3 is inflated, the air in esophagus is evacuated through the opening. After reaching a certain range of negative pressure, the traction balloon 4 is inflated to achieve the purpose of bending the esophagus. The opening can also be used to spray contrast agent, so that the curved part of the esophagus can be visualized under X-rays.

Specifically, when used for esophageal retraction, first insert the catheter into the esophagus through the nasal cavity or oral cavity without inflating the traction balloon and positioning balloon. After reaching the desired position, use the markers on the catheter and the handle to determine the bending direction as well as the position relative to the heart. Then the traction balloon is inflated and expanded, and the esophagus is retracted by means of the rigidity of the retractor. When it is necessary to rotate and adjust the positioning of the traction balloon on a horizontal section (when changing the ablation point for cardiac ablation), rotate the entire catheter through the knob on the handle to drive the traction balloon to rotate, thereby retracting the esophagus in a new direction to stay away from the new ablation points. The traction balloon is used to retract the part of esophagus between the second and third stenosis. The diameter of the positioning balloon is larger than the diameter of the esophagus at the stenosis. The length of the traction balloon is 10-15cm, and the working pressure is 2-8atm and the deviation distance 2-4cm.

## Claims

1. A curved balloon catheter retracting device **characterized by** comprising a catheter (5) with a handle (2) at one end, an inflatable positioner (3) positioned at a fixed location on the catheter, and a traction balloon (4) arranged outside the catheter adjacent the inflatable positioner and with upper and lower ends thereof being sealed on the catheter by laser welding or bonding, wherein one or a plurality of lumens (51, 52, 53, 54) are formed in the catheter, wherein the catheter is provided with at least one opening (511), wherein at least one lumen (51) is configured for inflating the traction balloon through the at least one opening, wherein the traction balloon is configured such that when the traction balloon is engaged within an interior lumen of the human body in a desired position and filled with fluid, it expands into an arc shape for engagement, retraction or displacement of tissue, and wherein the handle is configured to drive the catheter and the traction balloon to rotate, where the handle comprises a portion that is held stationary, and a rotatable angle portion provided with an angular dimension (21) indicated thereon, and when the handle is rotated, the handle is used to mark the relative azimuthal angle of the traction balloon.

2. The device of claim 1, wherein said inflatable positioner employs radiopaque material (31) configured for viewing the position of the inflatable positioner within the body by X-ray.

3. The device of claim 1, wherein the inflatable positioner is one or a plurality of balloons positioned at fixed locations on the catheter.

4. The device of claim 1, wherein the catheter has a length of scale for marking the depth of insertion of the catheter.

5. The device of claim 3, wherein one or a plurality of said lumens are used to fill the one or a plurality of balloons with fluid.

6. The device of claim 1, wherein at least one of said lumens is used for drainage of effusion.

7. The device of claim 6, wherein an opening (531) of the lumen (53) for drainage of effusion from the catheter is located near the proximal end of the traction balloon; and near the proximal end of the inflatable positioner.

8. The device of claim 1, wherein at least one of the lumens (54) is adapted to spray contrast agent; an opening (541) to spray the contrast agent is located near the proximal end of the traction balloon.

9. The device of claim 1, wherein the inflatable positioner is composed of two positioning balloons (3), which are respectively fixed on the catheter (5) at the proximal and distal end of the traction balloon (4), wherein at least one of said lumens (54) is adapted to receive a negative pressure, the opening (541) of which is located between the two positioning balloons at with one or a plurality of openings for drawing the negative pressure.

10. The device of claim 1, wherein said traction balloon is made of a material mixed with radiopaque materials so that it has the function of being detected under X-rays.

## Patentansprüche

1. gebogene Ballonkatheter-Rückzugsvorrichtung, **dadurch gekennzeichnet, dass** sie einen Katheter (5) mit einem Griff (2) an einem Ende, einen aufblasbaren Positionierer (3), der an einer festen Stelle auf dem Katheter positioniert ist, und einen Zugballon (4) umfasst, der außerhalb des Katheters angrenzend an den aufblasbaren Positionierer angeordnet ist und dessen oberes und unteres Ende auf dem Katheter durch Laserschweißen oder -bonden abgedichtet sind,
wobei ein oder eine Vielzahl von Lumen (51, 52, 53, 54) in dem Katheter ausgebildet sind, wobei der Katheter mit mindestens einer Öffnung (511) bereitgestellt ist, wobei mindestens ein Lumen (51) zum Aufblasen des Zugballons durch die mindestens eine Öffnung konfiguriert ist, wobei der Zugballon so konfiguriert ist, dass er sich, wenn der Zugballon in einem inneren Lumen des menschlichen Körpers in einer gewünschten Position eingreift und mit Flüssigkeit gefüllt ist, in eine Bogenform ausdehnt, um Gewebe in Eingriff zu bringen, zurückzuziehen oder zu verschieben, und wobei der Griff dazu konfiguriert ist, den Katheter und den Zugballon in Rotation zu versetzen, wobei der Griff einen Abschnitt, der stationär gehalten wird, und einen drehbaren Winkelabschnitt umfasst, der mit einer darauf angegebenen Winkelabmessung (21) bereitgestellt ist, und wobei der Griff bei Drehung dazu verwendet wird, den relativen Azimutwinkel des Zugballons zu markieren.

2. Vorrichtung nach Anspruch 1, wobei der aufblasbare Positionierer Kontrastmittel (31) verwendet, das dazu konfiguriert ist, die Position des aufblasbaren Positionierers innerhalb des Körpers mittels Röntgenstrahlen zu betrachten.

3. Vorrichtung nach Anspruch 1, wobei der aufblasbare Positionierer ein oder eine Vielzahl von Ballons ist, die an festen Stellen auf dem Katheter positioniert sind.

4. Vorrichtung nach Anspruch 1, wobei der Katheter eine Längenskala zum Markieren der Einführungstiefe des Katheters aufweist.

5. Vorrichtung nach Anspruch 3, wobei ein oder eine Vielzahl der Lumen zum Füllen des einen oder einer Vielzahl von Ballons mit Flüssigkeit verwendet werden.

6. Vorrichtung nach Anspruch 1, wobei mindestens eines der Lumen zum Ableiten von Erguss verwendet wird.

7. Vorrichtung nach Anspruch 6, wobei eine Öffnung (531) des Lumens (53) zum Ableiten von Erguss aus dem Katheter in der Nähe des proximalen Endes des Zugballons und in der Nähe des proximalen Endes des aufblasbaren Positionierers angeordnet ist.

8. Vorrichtung nach Anspruch 1, wobei mindestens eines der Lumen (54) zum Sprühen von Kontrastmittel angepasst ist; eine Öffnung (541) zum Sprühen des Kontrastmittels in der Nähe des proximalen Endes des Zugballons angeordnet ist.

9. Vorrichtung nach Anspruch 1, wobei der aufblasbare Positionierer aus zwei Positionierungsballons (3) besteht, die jeweils an dem Katheter (5) an dem proximalen und distalen Ende des Zugballons (4) befestigt sind, wobei mindestens eines der Lumen (54) zum Aufnehmen eines Unterdrucks ausgelegt ist, dessen Öffnung (541) zwischen den beiden Positionierungsballons mit einer oder einer Vielzahl von Öffnungen zum Abziehen des Unterdrucks angeordnet ist.

10. Vorrichtung nach Anspruch 1, wobei der Zugballon aus einem Material hergestellt ist, das mit Kontrastmitteln gemischt ist, sodass er unter Röntgenstrahlen erkannt werden kann.

## Revendications

1. Dispositif de rétraction de cathéter à ballonnet incurvé, **caractérisé en ce qu'**il comprend un cathéter (5) avec une poignée (2) à une extrémité, un positionneur gonflable (3) positionné à un emplacement fixe sur le cathéter, et un ballonnet de traction (4) disposé à l'extérieur du cathéter adjacent au positionneur gonflable et dont les extrémités supérieure et inférieure sont scellées sur le cathéter par soudage ou liaison au laser,
dans lequel une ou une pluralité de lumières (51, 52, 53, 54) sont formées dans le cathéter, dans lequel le cathéter est pourvu d'au moins une ouverture (511), dans lequel au moins une lumière (51) est configurée pour gonfler le ballonnet de traction à travers l'au moins une ouverture, dans lequel le ballonnet de traction est configuré de telle sorte que lorsque le ballonnet de traction est engagé à l'intérieur d'une lumière intérieure du corps humain dans une position souhaitée et rempli de fluide, il se dilate en une forme d'arc pour l'engagement, la rétraction ou le déplacement de tissu, et dans lequel la poignée est configurée pour entraîner le cathéter et le ballonnet de traction à tourner, où la poignée comprend une partie qui est maintenue stationnaire, et une partie d'angle rotative pourvue d'une dimension angulaire (21) indiquée sur celle-ci, et lorsque la poignée est tournée, la poignée est utilisée pour marquer l'angle azimutal relatif du ballonnet de traction.

2. Dispositif selon la revendication 1, dans lequel ledit positionneur gonflable utilise un matériau radio-opaque (31) configuré pour visualiser la position du positionneur gonflable à l'intérieur du corps par rayons X.

3. Dispositif selon la revendication 1, dans lequel le positionneur gonflable est un ou une pluralité de ballonnets positionnés à des emplacements fixes sur le cathéter.

4. Dispositif selon la revendication 1, dans lequel le cathéter a une longueur d'échelle pour marquer la profondeur d'insertion du cathéter.

5. Dispositif selon la revendication 3, dans lequel une ou une pluralité desdites lumières sont utilisées pour remplir l'un ou une pluralité de ballonnets avec un fluide.

6. Dispositif selon la revendication 1, dans lequel au moins une desdites lumières est utilisée pour le drainage de l'épanchement.

7. Dispositif selon la revendication 6, dans lequel une ouverture (531) de la lumière (53) pour le drainage de l'épanchement à partir du cathéter est située près de l'extrémité proximale du ballonnet de traction ; et près de l'extrémité proximale du positionneur gonflable.

8. Dispositif selon la revendication 1, dans lequel au moins une des lumières (54) est adaptée pour pulvériser un agent de contraste ; une ouverture (541) pour pulvériser l'agent de contraste est située près de l'extrémité proximale du ballonnet de traction.

9. Dispositif selon la revendication 1, dans lequel le positionneur gonflable est composé de deux ballonnets de positionnement (3), qui sont respectivement fixés sur le cathéter (5) à l'extrémité proximale et distale du ballonnet de traction (4), dans lequel au moins l'une desdites lumières (54) est adaptée pour recevoir une pression négative, dont l'ouverture (541) est située entre les deux ballonnets de positionnement avec une ou une pluralité d'ouvertures pour aspirer la pression négative.

10. Dispositif selon la revendication 1, dans lequel ledit ballonnet de traction est constitué d'un matériau mélangé à des matériaux radio-opaques de sorte qu'il a pour fonction d'être détecté sous des rayons X.
